# EUROPÄISCHE PATENTSCHRIFT

(11) **EP 2 044 968 B1**
(45) Veröffentlichungstag und Bekanntmachung des Hinweises auf die Patenterteilung: **07.04.2010**
(21) Anmeldenummer: 08156890.9
(22) Anmeldetag: 26.05.2008
(51) Int. Cl.: A61M 16/00, F04B 19/00, F28D 15/00

(54) **Flüssigkeitsverdampfer**
Fluid vaporiser
Evaporateur de liquide

(30) Priorität: 04.10.2007 DE 102007047415
(43) Veröffentlichungstag der Anmeldung: 08.04.2009
(73) Patentinhaber: Dräger Medical AG & Co. KG, 23558 Lübeck (DE)
(72) Erfinder: Radomski, Klaus, 23566 Lübeck (DE)
(74) Vertreter: Strauss, Steffen

(56) Entgegenhaltungen:
- EP-A- 1 662 140
- US-A- 4 396 055
- US-A1- 2007 107 879

## Beschreibung

Die Erfindung betrifft einen Flüssigkeitsverdampfer gemäß Anspruch 1 und ein Verfahren zur Erzeugung von Dampf einer zu verdampfenden Flüssigkeit gemäß Anspruch 16.

Aus der Beatmungstechnik sind Flüssigkeitsverdampfer, die zur Abgabe eines Anästhesiemittels oder als Beatmungsanfeuchter für einen Patienten dienen, bekannt.

Aus der US 2007/107879 ist ein Flüssigkeitsverdampfer mit einem Flüssigkeitsvorrat einer zu verdampfenden Flüssigkeit, einem Flüssigkeitszuführungskanal und einer an den Flüssigkeitszuführungskanal angrenzenden Verdampfungseinheit bekannt. Die zu verdampfende Flüssigkeit wird mit Kapillarkräften in einen Flüssigkeitszuführungskanal gefördert. Der Flüssigkeitsverdampfer weist dabei keine beweglichen Teile auf. Die Kapillarpumpe besteht aus einem porösen Material, insbesondere Glassinter oder Keramiksinter, das mit der zu verdampfenden Flüssigkeit in Verbindung steht. In dem von außen beheizten Flüssigkeitszuführungskanal wird das sich durch den Kapillareffekt bewegende Wasser verdampft. Im Flüssigkeitszuführungskanal ist dazu ein als Kapillardocht wirkendes, poröses Material angeordnet. Mit einer Regelung der Temperatur des von außen beheizten Flüssigkeitszuführungskanals kann eine bestimmte Menge an Dampf der zu verdampfenden Flüssigkeit erzeugt werden. Ein Nachteil der bekannten Vorrichtung besteht in der thermischen Trägheit, insbesondere in der verzögerten Bewegung der Flüssigkeit in dem Flüssigkeitszuführungskanal und der Dauer der Erwärmung des Heizungselementes und damit des Flüssigkeitszuführungskanals. Der bekannte Flüssigkeitsverdampfer eignet sich für eine kontinuierliche Bereitstellung von Dampf, die problemlos realisiert werden kann. Bei einer intermittierenden Dampfbereitstellung kann es aufgrund der vorstehend genannten Gründe zu Verzögerungen in der Bereitstellung von Dampf kommen.

Ein Flüssigkeitsverdampfer findet u.a. Anwendung mit einer Atemvorrichtung, insbesondere bei einem offenen Beatmungssystem. Eine Atemvorrichtung erzeugt in der Inspirationsphase des Patienten Atemluft. Bei einem offenen System gelangt die Ausatemluft der Exspirationsphase in die Umgebung. Die Atemluft der Inspirationsphase wird mit Feuchtigkeit angereichert. Somit wird nur Dampf in der Inspirationsphase benötigt.

Insbesondere bei der Anwendung des aus der DE 10 2005 054 344 B3 bekannten Flüssigkeitsverdampfers mit einer Atemvorrichtung führt eine kontinuierliche Dampfproduktion bei kleinen Atemfrequenzen und großen Tidalvolumina dazu, dass eine große Menge an Dampf während der Exspirationsphase des Patienten zwischengespeichert werden muss. Eine Zwischenspeicherung von Dampf ist jedoch nur schwer realisierbar, da ein erzeugter Dampf unter den relativ kalten Umgebungsbedingungen leicht kondensieren kann.

Von Vorteil wäre daher eine Dampfproduktion ausschließlich während einer Inspirationsphase. Ein Anfeuchter mit einer mechanischen Pumpe, wie er beispielsweise in der DE 198 08 590 B2 beschrieben ist, kann prinzipiell eine kurzfristige Dampfbereitstellung realisieren. An einer mechanischen Pumpe sind jedoch Verschleißteile vorhanden, die vom Anwender regelmäßig ausgetauscht werden müssen.

Aus der EP 1 662 140 A2 ist eine Pumpvorrichtung für einen Transport einer Tintenflüssigkeit in einem Druckanpassungskanal eines Druckers bekannt, wobei der Transport durch eine Veränderung des Benetzungswinkels der Tintenflüssigkeit erfolgt.

Ausgehend vom Stand der Technik besteht die Aufgabe der vorliegenden Erfindung darin, einen Flüssigkeitsverdampfer anzugeben, der eine schnelle Bereitstellung von Dampf realisiert und insbesondere keine mechanische Pumpe benötigt.

Diese Aufgabe wird durch einen Flüssigkeitsverdampfer und ein Verfahren zur Erzeugung von Dampf einer zu verdampfenden Flüssigkeit mit den Merkmalen der unabhängigen Ansprüche gelöst.

In den davon abhängigen Ansprüchen sind vorteilhafte Ausgestaltungen und Weiterbildungen der Erfindung angegeben.

Der erfindungsgemäße Flüssigkeitsverdampfer ist neben einem Flüssigkeitsvorrat einer zu verdampfenden Flüssigkeit, wenigstens einem Flüssigkeitszuführungskanal und einer an den wenigstens einen Flüssigkeitszuführungskanal angrenzenden Verdampfungseinheit mit wenigstens einer Arbeitselektrode und wenigstens einer im Abstand zu der wenigstens einen Arbeitselektrode angeordneten Gegenelektrode versehen, wobei ein durch eine elektrische Spannung zwischen der wenigstens einen Arbeitselektrode und der wenigstens einen Gegenelektrode erzeugtes elektrisches Feld eine Bewegung der zu verdampfenden Flüssigkeit durch den Flüssigkeitszuführungskanal bewirkt. Der erfindungsgemäße Aufbau des Flüssigkeitsverdampfers ermöglicht, dass die zu verdampfende Flüssigkeit mit Hilfe der Elektrokapillarität innerhalb des wenigstens einen Flüssigkeitszuführungskanals bewegt wird und dabei gleichzeitig von der Verdampfungseinheit in dem Flüssigkeitszuführungskanal oder anschließend an den Flüssigkeitszuführungskanal zum Verdampfen gebracht wird. Die wenigstens eine Arbeitselektrode bildet wenigstens einen Teil einer Wand des Flüssigkeitszuführungskanals.

Das Phänomen der Elektrokapillarität nutzt die Tatsache aus, dass die Oberflächenspannung von Flüssigkeiten durch die Erzeugung eines elektrischen Feldes verändert werden kann. Somit verändert sich auch der Kontaktwinkel zwischen der Flüssigkeit und der Wand des Flüssigkeitszuführungskanals. Es ist somit möglich, durch Reduzierung des Kontaktwinkels von über 90 Grad (hydrophober Zustand) auf einen Winkel unter 90 Grad (hydrophiler Zustand) die zu verdampfende Flüssigkeit in dem Flüssigkeitszuführungskanal bewegen zu lassen.

In vorteilhafter Weise ist dabei die wenigstens eine Arbeitselektrode mit einer hydrophoben Schicht ausgeführt. Die wenigstens eine Arbeitselektrode hat dabei keinen direkten Kontakt mit der zu verdampfenden Flüssigkeit. Ein Vorteil dieser Ausführung besteht darin, dass bei Anlegen einer elektrischen Spannung zwischen der wenigstens einen Arbeitselektrode und der Gegenelektrode keine Elektrolyse auftreten kann. Somit kann eine höhere Spannung zwischen der wenigstens einen Arbeitselektrode und der Gegenelektrode angelegt werden, im Vergleich zu einer Arbeitselektrode ohne hydrophobe Schicht. Auch können aggressive zu verdampfende Flüssigkeiten Anwendung finden.

In einer weiteren vorteilhaften Ausgestaltung besteht die hydrophobe Schicht der wenigstens einen Arbeitselektrode wenigstens teilweise aus Teflon. Teflon besitzt eine hohe Wärmebeständigkeit, so dass in dieser Ausführungsvariante das wenigstens eine Heizelement der Verdampfungseinheit in Längsrichtung des Flüssigkeitszuführungskanals angeordnet sein kann. Das Heizelement kann dabei den Flüssigkeitszuführungskanal umgeben.

In einer wiederum weiter bevorzugten Ausführungsform umgibt die wenigstens eine Arbeitselektrode vollständig wenigstens einen Teil des Flüssigkeitszuführungskanals. Dadurch kann in vorteilhafter Weise eine Bewegung der zu verdampfenden Flüssigkeit beschleunigt werden.

Ferner kann ein weiterer Flüssigkeitszuführungskanal mit einer weiteren Arbeitselektrode in dem erfindungsgemäßen Flüssigkeitsverdampfer vorgesehen sein. Die weitere Arbeitselektrode kann mit einer weiteren Gegenelektrode oder mit der Gegenelektrode der Arbeitselektrode des ersten Flüssigkeitszuführungskanals verbindbar sein.

Die Arbeitselektrode eines ersten Flüssigkeitszuführungskanals kann dabei oder in einer weiteren Ausführung des erfindungsgemäßen Flüssigkeitsverdampfers mit der Gegenelektrode und jede weitere Arbeitselektrode jedes weiteren Flüssigkeitszuführungskanals mit jeder weiteren Gegenelektrode separat mit Spannung versorgt werden. Mit entsprechend angeordneten Schaltelementen können die jeweiligen Arbeitselektroden mit den zugehörigen Gegenelektroden einzeln angesteuert werden. Dadurch kann kurzzeitig eine größere Menge an Dampf der zu verdampfenden Flüssigkeit erzeugt werden. Die jeweiligen Arbeitselektroden können aber auch von nur einer einzigen Spannungsversorgung versorgt werden, wobei mit Schaltelementen, welche zwischen der Spannungsversorgung und der jeweiligen Arbeitselektrode angeordnet sind, eine separate Zu- oder Abschaltung der jeweiligen Arbeitselektrode ermöglicht werden kann.

Die vorliegende Erfindung wird mit Bezug auf die beigefügten Zeichnungen detaillierter erläutert, wobei gleiche Bezugszeichen gleiche Strukturen bezeichnen.

In der Zeichnung gilt:
- Figur 1: eine schematische Schnittdarstellung des erfindungsgemäßen Flüssigkeitsverdampfers in einer ersten Ausführung,
- Figur 2: eine schematische Schnittdarstellung des erfindungsgemäßen Flüssigkeitsverdampfers mit einer den Flüssigkeitszuführungskanal umgebenden Arbeitselektrode,
- Figur 3: eine schematische Schnittdarstellung des erfindungsgemäßen Flüssigkeitsverdampfers in einer Ausführung mit mehreren Flüssigkeitszuführungskanälen,
- Figur 4: eine schematische Schnittdarstellung des erfindungsgemäßen Flüssigkeitsverdampfers in einer Ausführung mit porösem Material zwischen jeweils zwei Flüssigkeitszuführungskanälen
- Figur 5: eine schematische Schnittdarstellung des erfindungsgemäßen Flüssigkeitsverdampfers mit drei Flüssigkeitszuführungskanälen mit rechteckigem Querschnitt und
- Figur 6: eine schematische Schnittdarstellung des erfindungsgemäßen Flüssigkeitsverdampfers mit drei Flüssigkeitszuführungskanälen unterschiedlicher Querschnittsfläche.

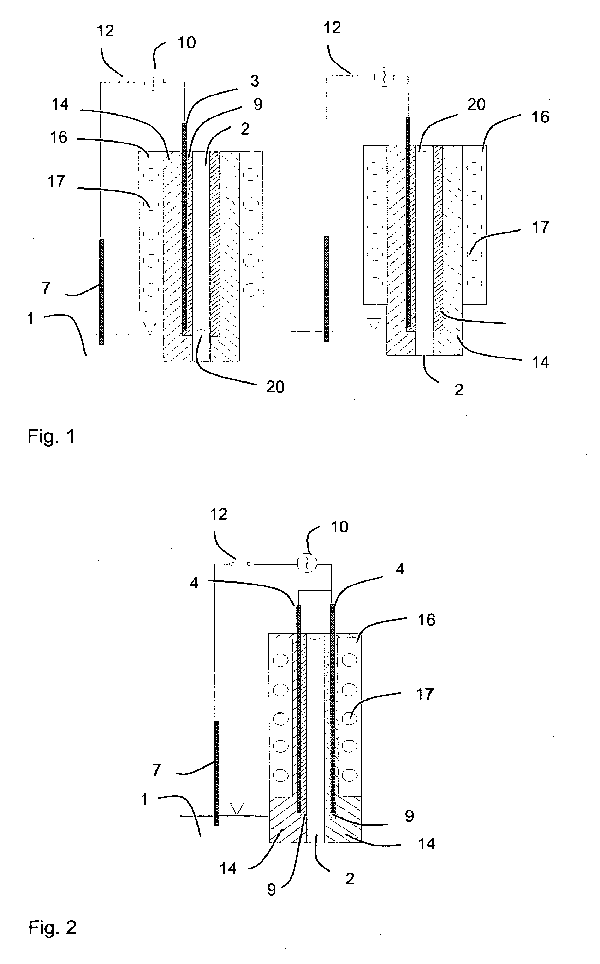

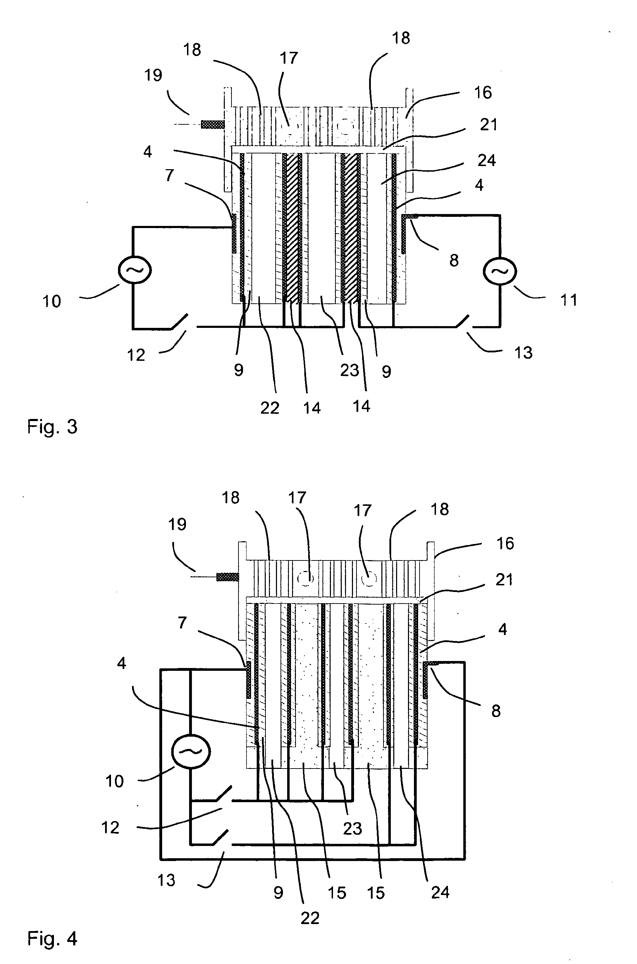

In der linken und rechten Darstellung der Figur 1 sind jeweils ein Flüssigkeitsverdampfer mit einer Arbeitselektrode 3 und einer im Abstand zur Arbeitselektrode 3 angeordneten Gegenelektrode 7 gezeigt. Die Arbeitselektrode 3 bildet einen Teil einer Wand eines Flüssigkeitszuführungskanals 2. Der Flüssigkeitszuführungskanal 2 ist mit einem Flüssigkeitsvorrat 1 einer zu verdampfenden Flüssigkeit verbunden. Die Oberfläche der Arbeitselektrode 4 ist mit einer hydrophoben Schicht 9 ausgebildet. Vorzugsweise besteht die hydrophoben Schicht 9 aus Teflon. Die hydrophobe Schicht 9 steht innerhalb des Flüssigkeitszuführungskanals 2 mit der zu verdampfenden Flüssigkeit des Flüssigkeitsvorrates 1 in Verbindung. In einer weiteren nicht dargestellten Ausführung kann die Arbeitselektrode 3 aber auch direkt mit der zu verdampfenden Flüssigkeit in Kontakt stehen. Vorzugsweise ist die der Arbeitselektrode 3 gegenüber angeordnete Wandseite des Flüssigkeitszuführungskanals 2 mit einer hydrophoben Schicht 9 ausgeführt. Die Arbeitselektrode 3 erstreckt sich nahezu über die gesamte axiale Länge des Flüssigkeitszuführungskanals 2 und wird von einer Trägereinheit 14 gehalten. Das Material der Trägereinheit 14 besteht vorzugsweise aus Silizium. An die Trägereinheit 14 grenzt in Umfangsrichtung eine Verdampfungseinheit 16, die fünf zueinander beabstandet angeordnete Heizelemente 17 umfasst. Die Verdampfungseinheit 16 ist in Längsrichtung des Flüssigkeitszuführungskanals 2 angeordnet. Die Verdampfungseinheit 16 kann in einer weiteren Ausführung (Figur 2) auch innerhalb der Trägereinheit 14 angeordnet sein. Der Flüssigkeitszuführungskanal 2 wird in der Ausführungen der Figur 1 direkt und in der Ausführung der Figur 2 indirekt von den Heizelementen 17 der Verdampfungseinheit 16 umgeben. In einer weiteren Ausführung können die Heizelemente 17 der Verdampfungseinheit 16 in Strömungsrichtung der zu verdampfenden Flüssigkeit, dem Flüssigkeitszuführungskanal 2 nachgeordnet angeordnet sein (in den Figuren 3 und 4 dargestellt).

Der Abstand zwischen der Arbeitselektrode 3 und der Gegenelektrode 7 ist derart gewählt, dass ein Anlegen einer elektrische Spannung einer Spannungsversorgung 10 durch ein Schaltelement 12 zwischen der Arbeitselektrode 3 und der Gegenelektrode 7 ein elektrisches Feld erzeugt, dass eine Bewegung der zu verdampfenden Flüssigkeit von dem Flüssigkeitsvorrat 1 in den Flüssigkeitszuführungskanal 2 bewirkt. Die elektrische Spannung der Spannungsversorgung 10 kann sowohl Gleichspannung als auch Wechselspannung sein. Die linke Darstellung der Figur 1 zeigt einen Flüssigkeitsstand 20 während eines geöffneten Zustands des Schaltelements 12. In der rechten Darstellung der Figur 1 ist der Flüssigkeitsstand 20 während eines geschlossenen Zustands des Schaltelements 12 dargestellt. Die in dem Flüssigkeitszuführungskanal 2 aufsteigende Flüssigkeit wird durch die von den Heizelementen 17 erzeugte Wärme im Flüssigkeitszuführungskanal 2 verdampft.

Der Durchmesser beziehungsweise die Breite des Flüssigkeitszuführungskanals 2 ist abhängig von der zwischen der Arbeitselektrode 3 und der Gegenelektrode 7 anliegenden elektrischen Spannung der Spannungsversorgung 10, der Schichtdicke und der Dielektrizitätskonstante der hydrophoben Schicht 9, der Länge des Flüssigkeitszuführungskanals 2, sowie von dem Abstand der Arbeitselektrode 3 von der Gegenelektrode 7 und der Art der zu verdampfenden Flüssigkeit. Elektrisch leitfähige Teilchen in der zu verdampfenden Flüssigkeit begünstigen die Bewegung der zu verdampfenden Flüssigkeit von dem Flüssigkeitsvorrat 1 in den Flüssigkeitszuführungskanal 2. Bei gleichen Kenngrößen des erfindungsgemäßen Flüssigkeitsverdampfers ist die Aufstiegsgeschwindigkeit eines Elektrolyts als zu verdampfende Flüssigkeit in dem Flüssigkeitszuführungskanal 2 höher im Vergleich mit entmineralisiertem Wasser oder destilliertem Wasser.

Bei einer Spannung der Spannungsversorgung 10 von beispielsweise 220 Volt, einer Länge des Flüssigkeitszuführungskanals 2 von 25 Millimetern, einer Schichtdicke der hydrophoben Schicht 9 von 18,5 Mikrometer, einer Dielektrizitätskonstante der hydrophoben Schicht 9 von 2,1 und einer zu verdampfenden Flüssigkeit eines entmineralisierten Wassers weist die Breite des Flüssigkeitszuführungskanals 2 cirka 100 Mikrometer auf.

Der Abstand zwischen Arbeitselektrode 3 und Gegenelektrode 7 liegt im Bereich von 1 bis 10 Millimetern.

Der in Figur 1 dargestellte erfindungsgemäße Flüssigkeitsverdampfer mit einer Breite des Flüssigkeitszuführungskanal 2 von 100 Mirkometern und einer Länge von 25 Millimeter kann eine Aufstiegsgeschwindigkeit der zu verdampfenden Flüssigkeit von 0,5 Millimeter pro Sekunde in einer Steighöhe von 25 Millimeter erzeugen. In experimentellen Untersuchungen konnte die Anmelderin eine Fördermenge der zu verdampfenden Flüssigkeit von ca. 0,8 Milliliter pro Minute ermitteln. Durch eine Verringerung der Schichtdicke der hydrophoben Schicht 9 und / oder mit einer hydrophoben Schicht 9 einer größeren Dielektrizitätskonstante kann die erreichbare Fördermenge der zu verdampfenden Flüssigkeit weiter gesteigert werden. Gleichzeitig kann bei einer identischen Fördermenge der zu verdampfenden Flüssigkeit die benötigte Spannung der Spannungsversorgung 10 signifikant reduziert werden kann.

Die schematische Schnittdarstellung der Figur 2 zeigt eine zweite Ausführung des erfindungsgemäßen Flüssigkeitsverdampfers mit einer den Flüssigkeitszuführungskanal 2 umgebenden Arbeitselektrode 4. Die Arbeitselektrode 4 umgibt den Flüssigkeitszuführungskanal 2 in dieser Ausführung vollständig. Dadurch wird eine elektrokapillare Wirkung verstärkt, da die Oberflächenspannung der zu verdampfenden Flüssigkeit bei Anliegen einer elektrischen Spannung zwischen der Arbeitselektrode 4 und der Gegenelektrode 7 von beiden Seiten reduziert wird. Die Arbeitselektrode 4 erstreckt sich, ebenso wie in der Darstellung der Figur 1, nahezu über die gesamte axiale Länge des Flüssigkeitszuführungskanals 2. Die Arbeitselektrode 4 weist ebenfalls eine hydrophobe Schicht 9 auf, kann aber auch in einer weiteren Ausführung direkt mit der zu verdampfenden Flüssigkeit in Kontakt stehen (nicht dargestellt). Ferner kann die Arbeitselektrode 4 in einer weiteren Ausführung nur einen Teil des Flüssigkeitszuführungskanals 2 vollständig umgeben, erstreckt sich jedoch auch in dieser Ausführung nahezu über die gesamte axiale Länge des Flüssigkeitszuführungskanals 2 (nicht dargestellt).

In der Figur 3 ist eine weitere Ausführungsvariante des erfindungsgemäßen Flüssigkeitsverdampfers mit mehreren Flüssigkeitszuführungskanälen dargestellt. Die Arbeitselektroden 4 sind gemäß der Beschreibung des Aufbaus der Arbeitselektrode 4 der Figur 2 ausgeführt. Die Arbeitselektrode 4 eines ersten Flüssigkeitszuführungskanals 22 ist mit einem Schalter 12 verbunden, der wahlweise die Arbeitselektrode 4 mit der Spannungsversorgung 10 verbindet. Die Spannungsversorgung 10 ist gleichzeitig mit der Gegenelektrode 7 verbunden. In einem an den ersten Flüssigkeitszuführungskanal 22 angrenzenden zweiten Flüssigkeitszuführungskanal 23 ist eine zweite Arbeitselektrode 4 vorgesehen, die mit der ersten Arbeitselektrode 4 des ersten Flüssigkeitszuführungskanals 22 elektrisch verbunden ist. Ein an den zweiten Flüssigkeitszuführungskanals 23 angrenzender dritter Flüssigkeitszuführungskanals 24 umfasst eine dritte Arbeitselektrode 4, die mit einem zweiten Schaltelement 13 verbunden ist und keine Verbindung zu den Arbeitselektroden 4 des ersten und zweiten Flüssigkeitszuführungskanals 22 und 23 aufweist. Das Schaltelement 13 verbindet wahlweise die Arbeitselektrode 4 mit einer Spannungsversorgung 11. Die Spannungsversorgung 11 ist gleichzeitig mit einer Gegenelektrode 8 verbunden. In dieser Ausführungsvariante können die Arbeitselektroden 4 des ersten und zweiten Flüssigkeitszuführungskanals 22 und 23 und die Gegenelektrode 7 durch das Schaltelement 12 wahlweise mit der Spannungsversorgung 10 verbunden werden. Hingegen kann die Arbeitselektrode 4 des dritten Flüssigkeitszuführungskanals 24 und die Gegenelektrode 8 separat mit elektrischer Spannung der Spannungsversorgung 11 versorgt werden. Mit dieser Anordnung kann eine zu erzeugende Menge an Dampf wahlweise erhöht oder reduziert werden, womit ein hoher Dynamikbereich des erfindungsgemäßen Flüssigkeitsverdampfers erzielt wird.

Die Verdampfungseinheit 16 ist in Strömungsrichtung der zu verdampfenden Flüssigkeit den Flüssigkeitszuführungskanälen 22, 23 und 24 nachgeordnet angeordnet. Die Heizelementen 17 sind quer zu den Flüssigkeitszuführungskanälen 22, 23 und 24 innerhalb der Verdampfungseinheit 16 angeordnet. In Längsrichtung der Flüssigkeitszuführungskanäle 22, 23 und 24 sind innerhalb der Verdampfungseinheit 16 einzelne Verdampfungskanäle 18 vorgesehen, um in vorteilhafter Weise eine großflächige Verdampfungsfläche zu realisieren. Vorzugsweise sind drei bis vier Verdampfungskanäle 18 in einzelnen Einheiten in unmittelbarer Nähe des Ausgangs der Flüssigkeitszuführungskanäle 22, 23 und 24 vorgesehen, wobei zwischen den Einheiten der Verdampfungskanäle 18 die Heizelemente 17 angeordnet sind. Die Flüssigkeitszuführungskanäle 22, 23 und 24 sind in vorteilhafter Weise durch ein Metallvlies 21 von den Verdampfungskanäle 18 getrennt. Damit kann sich die zu verdampfende Flüssigkeit in optimaler Weise von den Flüssigkeitszuführungskanälen 22, 23 und 24 auf die Verdampfungskanäle 18 ausbreiten. Ein Temperatursensor 19 ist in unmittelbarer Nähe der Verdampfungskanäle 18 angeordnet, um eine gemessene Temperatur zur Steuerung oder Kontrolle der Verdampfungseinheit 16 anzuwenden.

Die schematische Schnittdarstellung der Figur 4 zeigt den erfindungsgemäßen Flüssigkeitsverdampfer mit drei Flüssigkeitszuführungskanälen 22, 23 und 24, wobei zwischen dem ersten und dem zweiten Flüssigkeitszuführungskanal 22 und 23 und zwischen dem zweiten und dem dritten Flüssigkeitszuführungskanal 23 und 24 poröse Glassinterelemente 15 angeordnet sind. Die porösen Glassinterelemente 15 stehen in Kontakt mit der zu verdampfenden Flüssigkeit des Flüssigkeitsvorrates 1. Die porösen Glassinterelemente 15 wirken in dieser Anordnung als Docht, in denen die zu verdampfende Flüssigkeit aufgrund des Kapillareffektes von dem Flüssigkeitsvorrat 1 aufsteigt. Die Arbeitselektroden 4 sind grundsätzlich gemäß der Beschreibung des Aufbaus der Arbeitselektrode 4 der Figur 2 ausgeführt. Zudem weisen die Arbeitselektroden 4 auf der dem jeweiligen Flüssigkeitszuführungskanal 22, 23 und 24 abgewandten Seite eine zweite hydrophobe Schicht 9 auf. Die Arbeitselektrode 4 des Flüssigkeitszuführungskanals 22 ist sowohl mit einem Schalter 12 als auch mit einer zweiten Arbeitselektrode 4 eines an den ersten Flüssigkeitszuführungskanal 22 angrenzenden zweiten Flüssigkeitszuführungskanal 23 verbunden. Das Schaltelement 12 verbindet wahlweise beide Arbeitselektrode 4 mit der Spannungsversorgung 10. Die Spannungsversorgung 10 ist gleichzeitig mit den Gegenelektroden 7 und 8 elektrisch verbunden. Die Gegenelektroden 7 und 8 können auch in vorteilhafter Weise als eine gemeinsame Ringelektrode ausgeführt sein, die alle Flüssigkeitszuführungskanäle 22, 23 und 24 umfaßt. In einem an den zweiten Flüssigkeitszuführungskanal 23 angrenzenden dritten Flüssigkeitszuführungskanal 24 ist eine dritte Arbeitselektrode 4 vorgesehen, die mit einem Schaltelement 13 verbunden ist. Das Schaltelement 13 verbindet wahlweise die Arbeitselektrode 4 des dritten Flüssigkeitszuführungskanals 24 mit der Spannungsversorgung 10.
Sowohl die Arbeitselektroden 4 der ersten und zweiten Flüssigkeitszuführungskanäle 22 und 23 als auch die Arbeitselektrode 4 des dritten Flüssigkeitszuführungskanals 24 können wahlweise mit elektrischer Spannung der Spannungsversorgung 10 durch die Schaltelemente 12 und 13 versorgt werden.

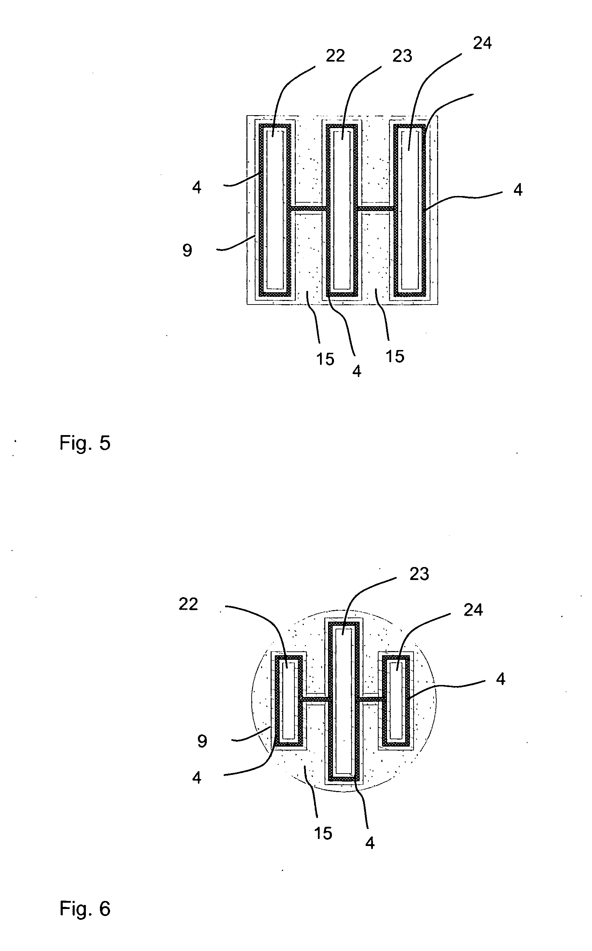

Mit dieser Anordnung kann eine Grundmenge an zu verdampfender Flüssigkeit mit den Glassinterelementen 15 bereitgestellt werden. Bei Bedarf kann die Menge an zu verdampfender Flüssigkeit kurzfristig durch das wahlweise Anlegen einer elektrischen Spannung der Spannungsversorgung 10 durch das Schaltelement 12 an den Arbeitselektroden 4 der ersten und zweiten Flüssigkeitszuführungskanäle 22 und 23 sowie durch das Schaltelement 13 der Arbeitselektrode 4 des dritten Flüssigkeitszuführungskanals 24 erhöht werden. Damit kann die zu erzeugende Menge an Dampf wahlweise über einer Grundmenge an Dampf erhöht werden, womit ein hoher Dynamikbereich des erfindungsgemäßen Flüssigkeitsverdampfers über der Grundmenge an Dampf erzielt wird. Eine schematische Schnittdarstellung des erfindungsgemäßen Flüssigkeitsverdampfers mit drei Flüssigkeitszuführungskanälen 22, 23 und 24 mit jeweils rechteckigem Querschnitt zeigt die Figur 5. In den einzelnen Flüssigkeitszuführungskanälen 22, 23 und 24 sind jeweils eine Arbeitselektrode 4 angeordnet, die grundsätzlich gemäß der Beschreibung des Aufbaus der Arbeitselektrode 4 der Figur 2 ausgeführt sind. Im Gegensatz zu den Ausführungen der Figuren 3 und 4 sind die Arbeitselektroden 4 der Ausführung der Figur 5 miteinander verbunden. Zwischen den einzelnen Flüssigkeitszuführungskanälen 22, 23 und 24 sind poröse Glaselemente 15 angeordnet. Eine elektrische Spannung wird zwischen den Arbeitselektroden 4 und einer oder mehrerer Gegenelektroden 7 und / oder 8 angelegt (nicht dargestellt).

Figur 6 zeigt eine schematische Schnittdarstellung des erfindungsgemäßen Flüssigkeitsverdampfers mit drei Flüssigkeitszuführungskanälen 22, 23 und 24 unterschiedlicher Querschnittsfläche. Die jeweils äußeren Flüssigkeitszuführungskanäle 22 und 24 weisen eine kleinere Querschnittsfläche im Vergleich zu dem inneren Flüssigkeitszuführungskanal 23 auf. Die einzelnen Flüssigkeitszuführungskanäle 22, 23 und 24 weisen wie in der Ausführung der Figur 5 jeweils eine Arbeitselektrode 4 auf. Die Arbeitselektroden 4 umschließen den jeweiligen Flüssigkeitszuführungskanal 22, 23 und 24 vollständig. Der erfindungsgemäße Flüssigkeitsverdampfer kann in dieser Ausführung eine runde Bauform aufweisen.

Mit dem erfindungsgemäßen Flüssigkeitsverdampfer kann eine schnelle Erzeugung von Dampf einer zu verdampfenden Flüssigkeit ohne die Verwendung verschleißbehafteter, mechanischer Bauteilen erreicht werden. Durch den erfindungsgemäßen Aufbau kann die Menge der zu verdampfenden Flüssigkeit aktiv kontrolliert und verändert werden. Insbesondere ist es mit dem erfindungsgemäßen Flüssigkeitsverdampfer in Zusammenwirkung mit einer Atemvorrichtung oder als Bestandteil einer Atemvorrichtung möglich, eine schnelle Dampferzeugung ausschließlich während der Inspirationsphase zu realisieren.

Während die vorliegende Erfindung unter Bezugnahme auf die bevorzugten Ausführungsbeispiele beschrieben worden ist, sind dem Fachmann verschiedene Änderungen und Modifikationen klar. All diese Änderungen und Modifikationen sollen in dem Schutzbereich der angefügten Ansprüche fallen.

### BEZUGSZEICHENLISTE

- 1: Flüssigkeitsvorrat
- 2, 22, 23, 24: Flüssigkeitszuführungskanal
- 3, 4: Arbeitselektrode
- 7,8: Gegenelektrode
- 9: Hydrophobe Schicht
- 10, 11: Spannungversorgung
- 12, 13: Schaltelement
- 14: Trägereinheit
- 15: Poröse Glassinterelemente
- 16: Verdampfungseinheit
- 17: Heizelement
- 18: Verdampfungskanal
- 19: Temperatursensor
- 20: Flüssigkeitsstand im Flüssigkeitszuführungskanal
- 21: Metallvlies

## Patentansprüche

1. Flüssigkeitsverdampfer mit einem Flüssigkeitsvorrat (1) einer zu verdampfenden Flüssigkeit, wenigstens einem Flüssigkeitszuführungskanal (2, 22, 23, 24) und einer an den wenigstens einen Flüssigkeitszuführungskanal (2, 22, 23, 24) angrenzenden Verdampfungseinheit (16),
**dadurch gekennzeichnet, dass**
wenigstens eine Arbeitselektrode (3, 4), die wenigstens einen Teil einer Wand des Flüssigkeitszuführungskanals (2, 22, 23, 24) bildet und wenigstens eine im Abstand zu der wenigstens einen Arbeitselektrode (3, 4) angeordnete Gegenelektrode (7, 8) vorgesehen sind, wobei ein durch eine elektrische Spannung zwischen der wenigstens einen Arbeitselektrode (3, 4) und der wenigstens einen Gegenelektrode (7, 8) erzeugtes elektrisches Feld eine Bewegung der zu verdampfenden Flüssigkeit durch den Flüssigkeitszuführungskanal (2, 22, 23, 24) bewirkt.

2. Flüssigkeitsverdampfer nach Anspruch 1, **dadurch gekennzeichnet, dass** der Flüssigkeitszuführungskanal (2, 22, 23, 24) einen rechteckigen, vorzugsweise quadratischen Querschnitt aufweist.

3. Flüssigkeitsverdampfer nach Anspruch 1, **dadurch gekennzeichnet, dass** der Flüssigkeitszuführungskanal (2, 22, 23, 24) einen runden Querschnitt aufweist.

4. Flüssigkeitsverdampfer nach einem der vorhergehenden Ansprüche, **dadurch gekennzeichnet, dass** ein Durchmesser beziehungsweise eine Breite des Flüssigkeitszuführungskanals (2, 22, 23, 24) in Abhängigkeit von einer an der wenigstens einen Arbeitselektrode (3, 4) und der wenigstens einen Gegenelektrode (7, 8) anliegenden Spannung ausgebildet ist und vorzugsweise einen Wert unter 200 Mikrometer aufweist.

5. Flüssigkeitsverdampfer nach einem der vorhergehenden Ansprüche, **dadurch gekennzeichnet, dass** ein Durchmesser beziehungsweise eine Breite des Flüssigkeitszuführungskanals (2, 22, 23, 24) in Abhängigkeit von dem Abstand der wenigstens einen Arbeitselektrode (3, 4) von der wenigstens einen Gegenelektrode (7, 8) ausgebildet ist und vorzugsweise einen Wert unter 200 Mikrometer aufweist

6. Flüssigkeitsverdampfer nach einem der vorhergehenden Ansprüche, **dadurch gekennzeichnet, dass** die wenigstens eine Arbeitselektrode (3, 4) von einer Trägereinheit (14) gehalten wird, wobei das Material der Trägereinheit (14) vorzugsweise aus Silizium besteht

7. Flüssigkeitsverdampfer nach einem der vorhergehenden Ansprüche, **dadurch gekennzeichnet, dass** die wenigstens eine Arbeitselektrode (3, 4) mit einer hydrophoben Schicht (9) ausgeführt ist, wobei die hydrophoben Schicht (9) vorzugsweise wenigstens teilweise aus Teflon besteht.

8. Flüssigkeitsverdampfer nach einem der vorhergehenden Ansprüche, **dadurch gekennzeichnet, dass** die Verdampfungseinheit (16) mit wenigstens einem Heizelement (17) ausgeführt ist, welches in Längsrichtung des Flüssigkeitszuführungskanals (2, 22, 23, 24) angeordnet ist und vorzugsweise den Flüssigkeitszuführungskanal (2, 22, 23, 24) umgibt.

9. Flüssigkeitsverdampfer nach einem oder mehreren der Ansprüche 1 bis 7, **dadurch gekennzeichnet, dass** die Verdampfungseinheit (16) mit wenigstens einem Heizelement (17) ausgeführt ist, welches in Strömungsrichtung der zu verdampfenden Flüssigkeit dem Flüssigkeitszuführungskanal (2, 22, 23, 24) nachgeordnet angeordnet ist.

10. Flüssigkeitsverdampfer nach einem der vorhergehenden Ansprüche, **dadurch gekennzeichnet, dass** die wenigstens eine Arbeitselektrode (3, 4) wenigstens einen Teil des Flüssigkeitszuführungskanals (2, 22, 23, 24) vollständig umgibt.

11. Flüssigkeitsverdampfer nach einem der vorhergehenden Ansprüche, **dadurch gekennzeichnet, dass** wenigstens in einem zweiten Flüssigkeitszuführungskanal (23, 24) eine weitere Arbeitselektrode (3, 4) vorgesehen ist, die mit der Arbeitselektrode (3, 4) des ersten Flüssigkeitszuführungskanals (22) verbunden ist.

12. Flüssigkeitsverdampfer nach einem oder mehreren der Ansprüche 1 bis 10, **dadurch gekennzeichnet, dass** in einem weiteren Flüssigkeitszuführungskanal (23, 24) eine weitere Arbeitselektrode (3, 4) vorgesehen ist, die mit einer weiteren Gegenelektrode (8) zusammen wirkt.

13. Flüssigkeitsverdampfer nach Anspruch 12, **dadurch gekennzeichnet, dass** Schaltelemente (12, 13) vorgesehen sind, so dass die wenigstens eine Arbeitselektrode (3, 4) mit der wenigstens einen Gegenelektrode (7) und jede weitere Arbeitselektrode (3, 4) mit jeder weiteren Gegenelektrode (8) separat mit Spannung versorgt werden können.

14. Flüssigkeitsverdampfer nach einem der vorhergehenden Ansprüche, **dadurch gekennzeichnet, dass** zwischen zwei Flüssigkeitszuführungskanälen (21, 22, 23) poröse Materialien angeordnet sind, vorzugsweise poröse Glas- oder Keramiksinterelemente (15).

15. Flüssigkeitsverdampfer nach einem der vorhergehenden Ansprüche, **dadurch gekennzeichnet, dass** die wenigstens eine Gegenelektrode (7, 8) außerhalb des Flüssigkeitszuführungskanals (2, 22, 23, 24) angeordnet ist.

16. Verfahren zur Erzeugung von Dampf einer zu verdampfenden Flüssigkeit umfassend die folgenden Schritte:
- zwischen wenigstens einer Arbeitselektrode (3, 4), die wenigstens einen Teil einer Wand eines Flüssigkeitszuführungskanals (2, 22, 23, 24) bildet und wenigstens einer im Abstand zu der wenigstens einen Arbeitselektrode (3, 4) angeordneten Gegenelektrode (7, 8) wird eine elektrische Spannung geschaltet, so dass zwischen der wenigstens einen Arbeitselektrode (3, 4) und der wenigstens einen Gegenelektrode (7, 8) ein elektrisches Feld erzeugt wird, wodurch eine zu verdampfende Flüssigkeit von einem Flüssigkeitsvorrat (1) in den Flüssigkeitszuführungskanal (2, 22, 23, 24) bewegt wird und
- die zu verdampfende Flüssigkeit an einer an dem Flüssigkeitszuführungskanal (2, 22, 23, 24) angrenzenden Verdampfungseinheit (16) verdampft wird.

## Claims

1. A liquid evaporator with a liquid reserve (1) of a liquid to be evaporated, at least one liquid feed duct (2, 22, 23, 24) and an evaporating unit (16) adjoining the at least one liquid feed duct (2, 22, 23, 24),
wherein
at least one working electrode (3, 4) forms at least one part of a wall of the liquid feed duct (2, 22, 23, 24) and at least one counterelectrode is provided, arranged at a spaced location from the at least one working electrode (3, 4)
wherein an electric field generated by an electric voltage between the at least one working electrode (3, 4) and the at least one counterelectrode (7, 8) brings about motion of the liquid to be evaporated through the liquid feed duct (2, 22, 23, 24).

2. A liquid evaporator in accordance with claim 1, wherein the liquid feed duct (2, 22, 23, 24) has a rectangular, preferably square cross section.

3. A liquid evaporator in accordance with claim 1, wherein the liquid feed duct (2, 22, 23, 24) has a round cross section.

4. A liquid evaporator in accordance with one of the preceding claims, wherein a diameter respectively a width of the liquid feed duct (2, 22, 23, 24) is selected as a function of a voltage to be applied to the at least one working electrode (3, 4) and the at least one counterelectrode (7, 8), preferably showing a value below 200 micrometers.

5. A liquid evaporator in accordance with one of the preceding claims, wherein a diameter respectively a width of the liquid feed duct (2, 22, 23, 24) is selected as a function of the distance between the at least one working electrode (3, 4) and the at least one counterelectrode (7, 8) and preferably shows a value below 200 micrometers.

6. A liquid evaporator in accordance with one of the preceding claims, wherein said at least one working electrode (3, 4) is held by a carrier unit (14), the material of said carrier unit (14) being preferably silicon.

7. A liquid evaporator in accordance with one of the preceding claims, wherein said at least one working electrode (3, 4) is provided with a hydrophobic layer, said hydrophobic layer (9) comprising Teflon at least partly.

8. A liquid evaporator in accordance with one of the preceding claims, wherein said evaporating unit (16) comprises at least one heating element (17) arranged in a longitudinal direction of said liquid feed duct (2, 22, 23, 24) and preferably surrounding said liquid feed duct (2, 22, 23, 24).

9. A liquid evaporator in accordance with one or several of the claims 1 to 7,
wherein said evaporating unit comprises at least one heating element (17) arranged downstream of said liquid feed duct (2, 22, 23, 24) in a direction of flow of the liquid to be evaporated.

10. A liquid evaporator in accordance with one of the preceding claims wherein said at least one working electrode (3, 4) completely surrounds at least one portion of said liquid feed duct (2, 22, 23, 24).

11. A liquid evaporator in accordance with one of the preceding claims, comprising at least an additional working electrode (3, 4) in an additional liquid feed duct (23, 24), said additional working electrode (3, 4) being connected to the working electrode (3, 4) of the first liquid feed duct (22).

12. A liquid evaporator in accordance with one or several of the claims 1 to 10,
wherein an additional working electrode (3, 4) is provided in an additional liquid feed duct (23, 24) cooperating with an additional counterelectrode (8).

13. A liquid evaporator in accordance with claim 12, comprising a switching arrangement (12, 13) for separately supplying voltage to the at least one working electrode (3, 4) with the at least one counterelectrode (7) and each additional working electrode (3, 4) with each additional counterelectrode (8).

14. A liquid evaporator in accordance with one of the preceding claims, wherein porous material, preferably porous glass or porous sintered ceramic elements (15) is arranged between two liquid feed ducts (21, 22, 23).

15. A liquid evaporator in accordance with one of the preceding claims, wherein the at least one counterelectrode (7, 8) is arranged outside of the liquid feed duct (2, 22, 23, 24).

16. Procedure for generating vapours in a liquid to be evaporated, comprising the following steps:
an electric voltage is applied between at least one working electrode (3,4), forming at least one part of a wall of a liquid feed duct (2,22, 23, 24) and at least one counterelectrode (7, 8) arranged at a spaced location from the at least one working electrode (3, 4), to generate an electric field between the at least one working electrode (3, 4) and the at least one counterelectrode (7, 8) for moving said liquid to be evaporated from a liquid reserve (1) into the liquid feed duct (2, 22, 23, 24) and
the liquid to be evaporated is evaporated at an evaporating unit (16) adjoining the liquid feed duct (2, 22, 23, 24).

## Revendications

1. Evaporateur de liquide comprenant une réserve (1) d'un liquide à évaporer, au moins un canal d'acheminement de liquide (2, 22, 23, 24) et une unité d'évaporation (16) adjacente au canal d'acheminement de liquide (2, 22, 23, 24), qui est au nombre minimum d'un,
**caractérisé en ce que**
sont prévus au moins une électrode de travail (3, 4) constituant au moins une partie d'une paroi du canal d'acheminement de liquide (2, 22, 23, 24) et au moins une contre-électrode (7, 8) située à distance de l'électrode de travail (3, 4), au nombre minimum d'une, un champ électrique généré par une tension électrique entre l'électrode de travail (3, 4), au nombre minimum d'une, et la contre-électrode (7, 8), au nombre minimum d'une, engendrant un déplacement du liquide à évaporer dans le canal d'acheminement de liquide (2, 22, 23, 24).

2. Evaporateur de liquide selon la revendication 1, **caractérisé en ce que** le canal d'acheminement de liquide (2, 22, 23, 24) présente une section transversale rectangulaire, de préférence carrée.

3. Evaporateur de liquide selon la revendication 1, **caractérisé en ce que** le canal d'acheminement de liquide (2, 22, 23, 24) présente une section transversale ronde.

4. Evaporateur de liquide selon l'une des revendications précédentes, **caractérisé en ce qu'**un diamètre et/ou une largeur du canal d'acheminement de liquide (2, 22, 23, 24) est déterminé(e) en fonction d'une tension présente au niveau d'une électrode de travail (3, 4), au nombre minimum d'une et de la contre-électrode (7, 8), au nombre minimum d'une, et présente, de préférence, une valeur inférieure à 200 micromètres.

5. Evaporateur de liquide selon l'une des revendications précédentes, **caractérisé en ce qu'**un diamètre et/ou une largeur du canal d'acheminement de liquide (2, 22, 23, 24) est déterminé(e) en fonction de la distance entre l'électrode de travail (3, 4), au nombre minimum d'une et la contre-électrode (7, 8), au nombre minimum d'une, et présente, de préférence, une valeur inférieure à 200 micromètres.

6. Evaporateur de liquide selon l'une des revendications précédentes, **caractérisé en ce que** l'électrode de travail (3, 4), au nombre minimum d'une, est maintenue par une unité formant support (14), le matériau constituant l'unité formant support (14) étant, de préférence, du silicium.

7. Evaporateur de liquide selon l'une des revendications précédentes, **caractérisé en ce que** l'électrode de travail (3, 4), au nombre minimum d'une, est pourvue d'une couche hydrophobe (9), la couche hydrophobe (9) étant de préférence constituée, au moins partiellement, en téflon.

8. Evaporateur de liquide selon l'une des revendications précédentes, **caractérisé en ce que** l'unité d'évaporation (16) est équipée d'au moins un élément chauffant (17) monté dans la direction longitudinale du canal d'acheminement de liquide (2, 22, 23, 24) et entourant de préférence le canal d'acheminement de liquide (2, 22, 23, 24).

9. Evaporateur de liquide selon une ou plusieurs des revendications 1 à 7, **caractérisé en ce que** l'unité d'évaporation (16) est équipée d'au moins un élément chauffant (17) monté, vu dans la direction d'écoulement du liquide à évaporer, en aval du canal d'acheminement du liquide (2, 22, 23, 24).

10. Evaporateur de liquide selon l'une des revendications précédentes, **caractérisé en ce que** l'électrode de travail (3, 4), au nombre minimum d'une, entoure complètement au moins une partie du canal d'acheminement de liquide (2, 22, 23, 24).

11. Evaporateur de liquide selon l'une des revendications précédentes, **caractérisé en ce qu'**au moins dans un deuxième canal d'acheminement de liquide (23, 24) est prévue une électrode de travail (3, 4) supplémentaire, qui est reliée à l'électrode de travail (3, 4) du premier canal d'acheminement de liquide (22).

12. Evaporateur de liquide selon une ou plusieurs des revendications 1 à 10, **caractérisé en ce que**, dans un canal d'acheminement de liquide (23, 24) supplémentaire, est prévue une électrode de travail (3, 4) supplémentaire, qui coopère avec une contre-électrode (8) supplémentaire.

13. Evaporateur de liquide selon la revendication 12, **caractérisé en ce que** sont prévus des éléments de commutation (12, 13) de telle sorte que l'électrode de travail (3, 4), au nombre minimum d'une, et la contre-électrode (7), au nombre minimum d'une, puissent être alimentées séparément en tension, ainsi que toute électrode de travail (3, 4) et que toute contre-électrode (8) supplémentaires.

14. Evaporateur de liquide selon l'une des revendications précédentes, **caractérisé en ce qu'**entre deux canaux d'acheminement de liquide (21, 22, 23) sont disposés des matériaux poreux, de préférence des éléments frittés poreux en verre ou en céramique (15).

15. Evaporateur de liquide selon l'une des revendications précédentes, **caractérisé en ce que** la contre-électrode (7, 8), au nombre minimum d'une, est disposée en-dehors du canal d'acheminement de liquide (2, 22, 23, 24).

16. Procédé de production de vapeur à partir d'un liquide à évaporer, comprenant les étapes suivantes :
- entre au moins une électrode de travail (3, 4) constituant au moins une partie d'une paroi d'un canal d'acheminement de liquide (2, 22, 23, 24) et au moins une contre-électrode (7, 8) située à distance de l'électrode de travail (3, 4), au nombre minimum d'une, est appliquée une tension électrique afin de générer un champ électrique entre l'électrode de travail (3, 4), au nombre minimum d'une, et la contre-électrode (7, 8), au nombre minimum d'une, ce qui engendre un déplacement du liquide à évaporer depuis une réserve de liquide (1) vers le canal d'acheminement de liquide (2, 22, 23, 24) et
- le liquide à évaporer est évaporé au niveau d'une unité d'évaporation (16) adjacente au canal d'acheminement de liquide (2, 22, 23, 24).
